# EUROPEAN PATENT SPECIFICATION

(11) **EP 1 549 444 B1**
(45) Date of publication and mention of the grant of the patent: **21.10.2015**
(21) Application number: 03753696.8
(22) Date of filing: 24.09.2003
(51) Int. Cl.: B08B 9/043, B08B 9/055, B24D 99/00, F16L 55/26, F16L 55/28

(54) **APPARATUS FOR CLEANING THE SURFACES OF BORES**
VORRICHTUNG ZUM REINIGEN DER FLÄCHEN VON BOHRUNGEN
APPAREIL PERMETTANT DE NETTOYER LES SURFACES D'UN PUITS DE FORAGE

(30) Priority: 24.09.2002 GB 0222136
(43) Date of publication of application: 06.07.2005
(73) Proprietor: Industrial Brushware Limited, Halesowen B62 8JJ (GB)
(72) Inventor: Palmer, Horace Dana, Barnt Green, Worcestershire B45 8JL (GB); Palmer, Jane Alison, Barnt Green, Worcestershire B45 8JL (GB)
(74) Representative: Somervell, Thomas Richard
(86) International application number: PCT/GB2003/004151
(87) International publication number: WO 2004/028714

(56) References cited:
- DE-C- 579 919
- FR-A- 643 717
- GB-A- 2 333 341
- GB-A- 2 342 372
- NL-A- 8 802 767
- US-A- 5 802 667

## Description

This invention relates to apparatus for cleaning the surfaces of bores by moving along inside them.

The invention is primarily, but not exclusively, concerned with the cleaning of bores which convey fluids, that is to say liquids or gases.

The invention has, in fact, been developed in connection with the cleaning of the internal surfaces of pipelines, particularly oil pipelines (although the invention is also applicable to the cleaning of water pipelines). The following description of the invention will therefore be confined mainly to this application. However, it is to be understood that there is no limitation in this regard as the invention has a wide range of other possible uses, the term "cleaning" being intended to include scouring, polishing, abrading, scraping, deburring and reaming and other similar operations, and therefore the invention is applicable to any apparatus for performing such operations in any appropriate circumstances in which the surfaces of bores or the internal surfaces of tubes or pipes require to be cleaned. In this respect it is believed that the invention may even be applicable to the debriding of human blood vessels to alleviate heart disease.

Apparatus for cleaning the internal surfaces of oil pipelines, which are commonly referred to as "pigs", are propelled along a pipeline by the flow of oil through it and clean the internal surface of the pipeline as they travel along it. Known forms of such apparatus have certain limitations. They do not achieve optimum cleaning and they frequently become jammed in pipelines causing a considerable amount of work and expense to release them. Also, cleaning a pipeline often requires the use initially of a gauging pig to check the internal diameter of the pipe, a number of cleaning pig runs depending on the frequency of cleaning, and then the use of an inspection pig to check the state of the pipeline.

FR 643 717 A1 discloses an apparatus in accordance with the preamble of claim 1 for cleaning a bore comprising a flexible cage that engages the inner surface of the bore. The cage is mounted on a rigid rod that passes through the cage by means of a mounting member at either end. Each mounting member is mounted to the rod by means of a screw thread so that the spacing between the members can be adjusted to vary the outer diameter of the cage.

The present invention has for its object the provision of an improved form of apparatus for cleaning the surfaces of bores by moving along inside them. A particular aim of the invention is to provide an apparatus for cleaning the internal surfaces of pipelines which is more effective and less likely to become jammed than existing pigs. A further aim of the invention is to provide an apparatus for cleaning the internal surfaces of pipelines which, as a result of its improved cleaning efficiency, is capable of cleaning a pipe with a minimum number of runs.

According to the invention there is provided an apparatus for cleaning the surface of a bore which comprises one or more cleaning elements for contact with the surface of a bore as the apparatus mover along inside the bore, and means operable to move the cleaning element or elements inwardly and outwardly to cater for bores of different sizes; wherein said means comprises an elongate resiliently deformable member of helical or substantially helical form which is arranged to effect said inward and outward movement of said cleaning elements by twisting, extending or contracting of at least a portion of the member resulting in an increase or decrease in the diameter of the at least a portion of the member and a support extending through the member with the ends of the member connected to it, the support comprising means permitting relative turning movement in opposite directions about the axis of the member between the ends of the member in order to vary the axial spacing between the ends of the member so as to selectively vary the diameter of the at least a portion of the member, characterised in that the support means includes a coil spring which is of a smaller diameter than and disposed concentrically within the member, the member and the coil spring being of opposite hands, whereby the member can bend as the apparatus moves through a bore.

Preferably the member is constituted by a coil spring.

The cleaning element or elements is or are conveniently mounted on the periphery of the member. The, or each, cleaning element is preferably in the form of a brush although elements of other forms may be used for different cleaning requirements. The apparatus may have one continuous cleaning element or a number of separate elements, for example in the form of a strip or strips. The element or elements may extend along the elongate member in a helical or substantially helical arrangement or there they may be a number of elements arranged in one or more circles perpendicular to the axis of the apparatus or in one or more ellipses at an angle to said axis.

The cleaning element or elements may be fixed to the member or movable inwardly and outwardly relative to it. In the latter event, means are preferably provided for moving the cleaning element(s) in this way. Such means may be inflatable means arranged by inflation and deflation thereof to move the cleaning element(s) or a bellows arranged by expansion and contraction thereof to move the cleaning element(s). Another form of such means may comprise springs and releasable means operable to hold the springs in compression with the cleaning element or elements in an inward retracted position and arranged when released to move the cleaning element(s) outwardly for contact with the surface of a bore to be cleaned.

The member may be designed to be twisted, extended or contracted by hand to set it at the required diameter for the bore to be cleaned, locking means being provided for locking the member in position. Alternatively, or in addition, the apparatus may be provided with power-operated means for twisting, extending or contracting the member. These means may be an electric, pneumatic or hydraulic motor or a hydraulic or pneumatic piston and cylinder unit. By making the power-operated means operable by remote-control the position of the cleaning element or elements can be adjusted *in situ,* not only to set up the apparatus but also to release it should it become jammed.

In a preferred embodiment the support comprises two coaxial components attached respectively to the ends of the coil spring and provided with cooperating screw threads permitting relative movement of the components and twisting of the member by hand but preventing such movement under the torque exerted by the member thereby to provide the aforesaid locking means. Where power-operated means are provided these may comprise a bellows arranged by expansion and contraction thereof to twist the member and thereby vary its diameter, or a gearing driven by a motor and arranged to turn the two components relative to one another.

The apparatus is preferably designed to pass through bends of up to 90° in a bore. The apparatus may be adjustable in size so that it can be made smaller for sharp bends and larger for large bends. In this way the efficiency of the apparatus can be maintained irrespective of the geometry of the bore to be cleaned.

The apparatus may further include means such as a piston and cylinder unit operable to expand the member axially beyond its normal range of expansion and thereby reduce its diameter to such an extent that the apparatus can be unjammed from relatively large obstructions.

According to an important feature of the invention the apparatus may be provided with means operable to obtain data relating to a bore and/or to the operation of the apparatus as it travels through the bore. Preferably these means are adapted to transmit such data to a remote receiver in real time or/and the data may be downloaded at the end of a run. In the case of an apparatus for cleaning the internal surfaces of pipelines this obviates the need for a separate inspection pig. Also, the number of separate runs required to clean a pipeline is reduced.

These means may be adapted to examine the internal surface of the bore, measure the distance travelled by the apparatus, measure the thickness of deposits on the surface, detect corrosion in the pipeline, measure the diameter of the cleaned surface and communicate in real time to provide information on the current state of the pipeline including measuring the oil pressure which provides an indication of the cleanliness of the pipeline.

The said means may be adapted to control the operation of the apparatus either automatically or under remote control to adjust the position of the cleaning elements during a run.

The apparatus may be provided with vanes or other formations to enhance the propulsion of the apparatus through a pipe under the impetus of oil flowing through it. Preferably these formations are also adapted to rotate the apparatus about an axis coincident with the pipe axis.

The invention will now be described, by way of example, with reference to the accompanying drawings, in which:-
FIGURE 1 is a side view of one form of apparatus for cleaning the internal surface of a pipe according to the invention,
FIGURE 2 is an end view of the apparatus,
FIGURE 3 is a section taken on the line A-A of FIGURE 2,
FIGURE 4 is a partial view showing in detail the way in which the brush is attached to the coil spring of the apparatus,
FIGURE 5 is an enlargement of part of FIGURE 4,
FIGURES 6A, 6B and 6C (referred to collectively as FIGURE 6) show diagrammatically one arrangement for moving the brush in a modification of the apparatus,
FIGURES 7A, 7B and 7C (referred to collectively as FIGURE 7) are diagrammatic illustrations of another arrangement for moving the brush and
FIGURE 8 is a perspective view showing part of another form of brush which may be used in the apparatus.

The drawings show an apparatus for cleaning the internal surface of a pipe which is designed to be used primarily as a pig for cleaning the internal surface of an oil pipeline.

The apparatus comprises a support generally designated 10 carrying a coil spring 11 which in turn carries a continuous brush 12 for cleaning the internal surface of a pipe as the apparatus travels along inside pipe, the apparatus being propelled by the flow of oil through the pipe.

The support comprises two circular end assemblies 13, 14 secured coaxially to the respective ends of the spring 11. The end assembly 13 includes a coaxial cylindrical component 15 the outer end portion of which is formed with a screw thread 15a and extends into a complementarily screw threaded bore 16a in a further cylindrical component 16. This component 16 is connected by a coil spring 17 to a further cylindrical component 18 which forms a coaxial part of the end assembly 14. The ends of the spring 17 embrace the opposed end portions of the components 16 and 18. The springs 11 and 17 are of opposite hands.

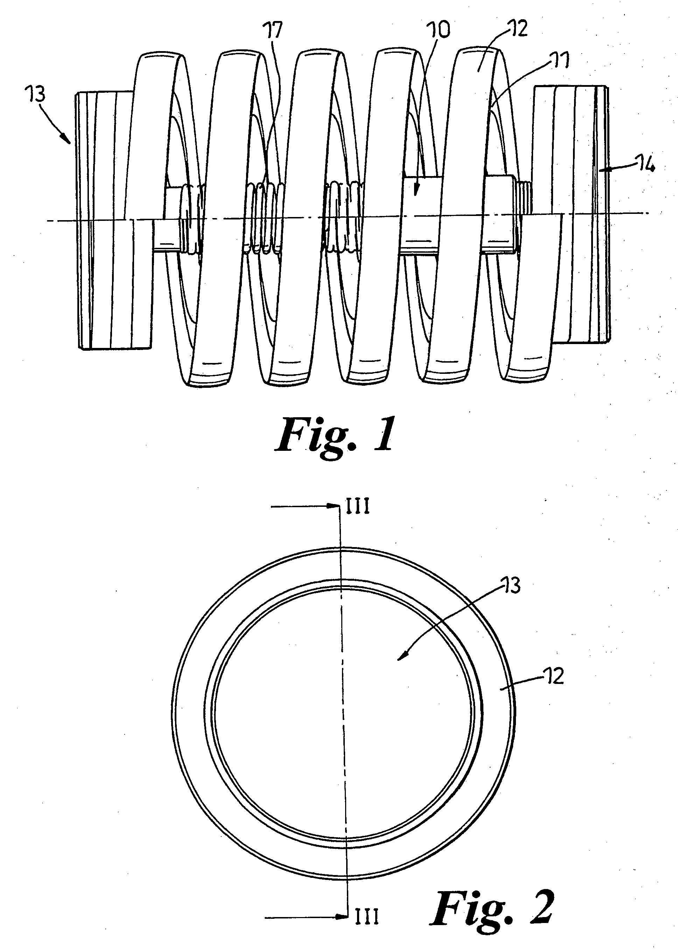

FIGURES 1, 2 and 3 illustrate diagrammatically the brush 12 attached to the spring 11. The brush is in the form of a continuous strip of bristles extending along the periphery of the spring 11 along the whole length of the spring.

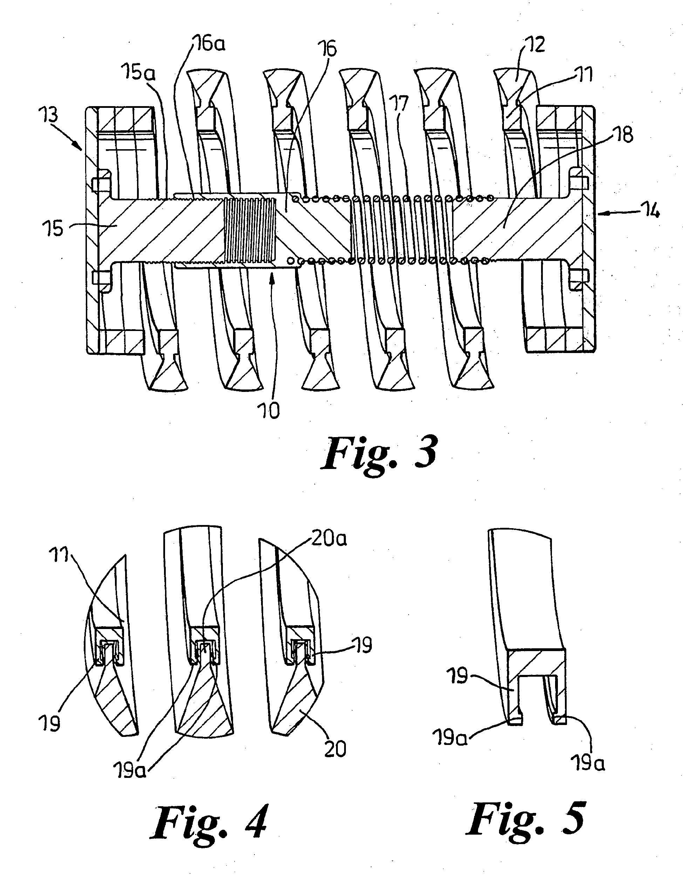

FIGURES 4 and 5 of the drawings show one method by which the brush may be attached to the spring. The outer periphery of the spring is formed with a continuous outwardly facing channel 19 having two inwardly extending lips 19a at the mouth of the channel. The brush 20 has an inner portion 20a which is received in the channel 19, being of outwardly tapering form towards the base of the channel so that it is held in position by the lips 19a.

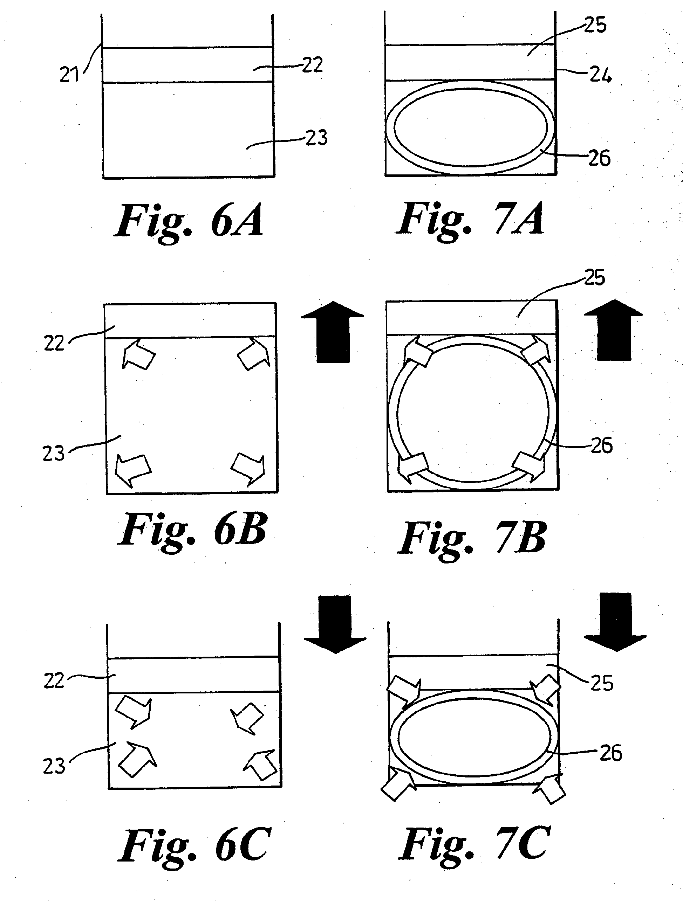

In a modification means may be provided for moving the brushes inwardly and outwardly relative to the spring 11. Two ways of doing this are illustrated diagrammatically in FIGURES 6 and 7.

In FIGURE 6 the outer periphery of the spring 11 is formed with an outwardly facing channel 21 and mounted for sliding movement inwardly and outwardly in this channel is a base 22 of a brush. The brush base and the channel form a sealed void which is filled with liquid or gas 23. This void is connected to a reservoir of the fluid or gas through a pump which is operable to supply more fluid or gas to the void so that the increased pressure inside the void as represented by the arrows in FIGURE 6B moves the brush base outwardly. Similarly reducing the pressure as represented by the arrows in FIGURE 6C pulls the brush base inwardly.

FIGURE 7 shows another arrangement which is similar to that shown in FIGURE 6 insofar as it comprises a channel 24 formed in the spring 11 and a brush base 25 but in this case the void contains an inflatable bag or sac 26. The supply of fluid or gas to this bag and removal of the pressure cause the brush to move outwardly and inwardly relative to the spring 11.

In use, the apparatus is first set by hand to a position dependent on the diameter of the pipe to be cleaned. This is done by screwing the component 15 into or out of the component 16 which has the effect of twisting the spring 11 and thereby increasing or reducing its diameter. The apparatus then remains in this position as the screw thread prevents relative turning of the components 15 and 16. In the modification of FIGURE 6 or 7 the position of the brush can also be adjusted so that as the apparatus passes along a pipe the brush sweeps its internal surface and thereby cleans it. If the apparatus encounters any bends or obstructions it can pass round these as the spring 17 allows the spring 11 to bend.

The apparatus may be provided with vanes or other formations to enhance the propulsion of the apparatus through a pipe and to rotate the apparatus about the axis of the pipe under the impetus of oil flowing through it.

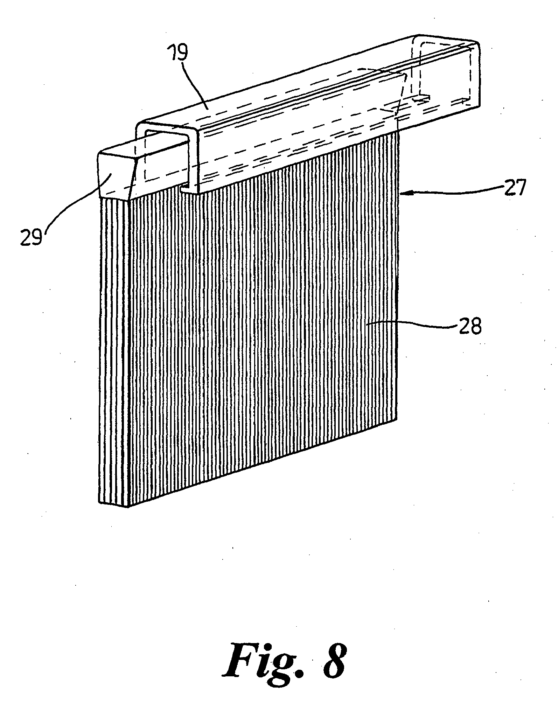

FIGURE 8 shows another form of brush 27 which may be used in the apparatus illustrated in FIGURES 1 to 3. This brush, like the brush 12, comprises a continuous strip of bristles 28 but in this case the bristles are longer. The base of the strip of bristles is attached to a base member 29 which is secured in the channel 19 carried by the spring 11.

## Claims

1. An apparatus for cleaning the surface of a bore which comprises one or more cleaning elements (12; 27) for contact with the surface of a bore as the apparatus mores along the bore, and means operable to move the cleaning element or elements inwardly and outwardly to cater for bores of different sizes; wherein said means comprises an elongate resiliently deformable member (11) of helical or substantially helical form which is arranged to effect said inward and outward movement of said cleaning elements by twisting, extending or contracting of at least a portion of the member resulting in an increase or decrease in the diameter of the at least a portion of the member and a support (10) extending through the member with the ends of the member connected to it, the support comprising means (13, 14, 15, 16) permitting relative turning movement in opposite directions about the axis of the member between the ends of the member (11) in order to vary the axial spacing between the ends of the member so as to selectively vary the diameter of the at least a portion of the member, **characterised in that** the support means (10) includes a coil spring (17) which is of a smaller diameter than and disposed concentrically within the member (11), the member and the coil spring being of opposite hands, whereby the member can bend as the apparatus moves through a bore.

2. An apparatus as claimed in claim 1, in which the member (11) is constituted by a coil spring.

3. An apparatus as claimed in claim 1 or claim 2, in which the cleaning element or elements (12; 27) is or are mounted on the periphery of the member (11).

4. An apparatus as claimed in any one of the preceding claims, in which the, or each, cleaning element (12; 27) is in the form of a brush.

5. An apparatus as claimed in any one of the preceding claims which has one continuous cleaning element (12; 27).

6. An apparatus as claimed in any one of the preceding claims, in which the cleaning element or elements (12; 27) extends or extend along the elongate member (11) in a helical or substantially helical arrangement.

7. An apparatus as claimed in any one of the preceding claims, in which the cleaning element or elements (12; 27) is or are fixed to the member (11).

8. An apparatus as claimed in any one of claims 1 to 6, in which the cleaning element or elements (12) is or are mounted for movement inwardly and outwardly relative to the member (11) and means (21, 22, 23; 24, 25, 26) are provided for effecting such movement.

9. An apparatus as claimed in any one of the preceding claims, in which the means (13, 14, 15, 16) permitting relative turning movement in opposite directions about the axis of the member (11) between the ends of the member is arranged to be operated manually to set the at least a portion of the member (11) at a predetermined diameter, locking means (15a, 16a) being provided for locking the member in position.

10. An apparatus as claimed in any one of claims 1 to 8, in which the means (13, 14, 15, 16) permitting relative turning movement in opposite directions about the axis of the member (11) between the ends of the member is provided with power-operated means for twisting, extending or contracting the at least a portion of the member (11).

11. An apparatus as claimed in claim 9, in which the support (10) comprises two coaxial components (15, 16) attached respectively to the ends of the member (11) and provided with cooperating screw threads (15a, 16a) permitting relative movement of the components and twisting of the member by hand but preventing such movement under the torque exerted by the member thereby to provide the aforesaid locking means (15a, 16a).

12. An apparatus as claimed in any one of the preceding claims which is provided with means operable to obtain data relating to a bore and/or to the operation of the apparatus as it travels through the bore.

13. An apparatus as claimed in any of the preceding claims which is provided with vanes arranged, in use, to propel the apparatus through a bore and to rotate the apparatus about its axis under the impetus of fluid flowing through the bore.

## Patentansprüche

1. Vorrichtung zum Reinigen der Fläche einer Bohrung, die aufweist: ein oder mehrere Reinigungselemente (12; 27) für einen Kontakt mit der Fläche einer Bohrung, während sich die Vorrichtung längs der Bohrung bewegt; und ein Mittel, das funktionsfähig ist, um das Reinigungselement oder die Reinigungselemente nach innen und nach außen zu bewegen, um Bohrungen von unterschiedlichen Größen zu versorgen; wobei das Mittel aufweist: ein längliches elastisch verformbares Element (11) von spiralförmiger oder im Wesentlichen spiralförmiger Form, das angeordnet ist, um die Bewegung der Reinigungselemente nach innen und nach außen durch Verdrehen, Ausdehnen oder Zusammenziehen von mindestens einem Abschnitt des Elementes zu bewirken, was zu einer Vergrößerung oder Verkleinerung des Durchmessers des mindestens einen Abschnittes des Elementes führt; und eine Auflage (10), die sich durch das Element erstreckt, wobei die Enden des Elementes damit verbunden sind, wobei die Auflage ein Mittel (13, 14, 15, 16) aufweist, das eine relative Drehbewegung in entgegengesetzten Richtungen um die Achse des Elementes zwischen den Enden des Elementes (11) gestattet, um den axialen Abstand zwischen den Enden des Elementes zu verändern, um so selektiv den Durchmesser des mindestens einen Abschnittes des Elementes zu verändern, **dadurch gekennzeichnet, dass** das Auflagemittel (10) eine Schraubenfeder (17) umfasst, die einen kleineren Durchmesser als das Element (11) aufweist und konzentrisch innerhalb dieses angeordnet ist, wobei das Element und die Schraubenfeder von entgegengesetzter Richtung sind, wobei sich das Element biegen kann, während sich die Vorrichtung durch eine Bohrung bewegt.

2. Vorrichtung nach Anspruch 1, bei der das Element (11) durch eine Schraubenfeder gebildet wird.

3. Vorrichtung nach Anspruch 1 oder Anspruch 2, bei der das Reinigungselement oder die Reinigungselemente (12; 27) am Umfang des Elementes (11) montiert ist oder sind.

4. Vorrichtung nach einem der vorhergehenden Ansprüche, bei der das oder jedes Reinigungselement (12; 27) in der Form einer Bürste vorliegt.

5. Vorrichtung nach einem der vorhergehenden Ansprüche, die ein kontinuierliches Reinigungselement (12; 27) aufweist.

6. Vorrichtung nach einem der vorhergehenden Ansprüche, bei der sich das Reinigungselement oder die Reinigungselemente (12; 27) längs des länglichen Elementes (11) in einer spiralförmigen oder im Wesentlichen spiralförmigen Anordnung erstreckt oder erstrecken.

7. Vorrichtung nach einem der vorhergehenden Ansprüche, bei der das Reinigungselement oder die Reinigungselemente (12; 27) am Element (11) befestigt ist oder sind.

8. Vorrichtung nach einem der Ansprüche 1 bis 6, bei der das Reinigungselement oder die Reinigungselemente (12) für eine Bewegung nach innen und nach außen relativ zum Element (11) montiert ist oder sind und Mittel (21, 22, 23; 24, 25, 26) für das Bewirken einer derartigen Bewegung vorhanden sind.

9. Vorrichtung nach einem der vorhergehenden Ansprüche, bei der das Mittel (13, 14, 15, 16), das die relative Drehbewegung in entgegengesetzten Richtungen um die Achse des Elementes (11) zwischen den Enden des Elementes gestattet, so ausgebildet ist, dass es manuell betätigt werden kann, um den mindestens einen Abschnitt des Elementes (11) auf einen vorgegebenen Durchmesser einzustellen, wobei ein Verriegelungsmittel (15a, 16a) für das Verriegeln des Elementes in Position vorhanden ist.

10. Vorrichtung nach einem der Ansprüche 1 bis 8, bei der das Mittel (13, 14, 15, 16), das die relative Drehbewegung in entgegengesetzten Richtungen um die Achse des Elementes (11) zwischen den Enden des Elementes gestattet, mit einem kraftbetätigten Mittel für das Verdrehen, Ausdehnen oder Zusammenziehen des mindestens einen Abschnittes des Elementes (11) versehen ist.

11. Vorrichtung nach Anspruch 9, bei der die Auflage (10) zwei koaxiale Bauteile (15, 16) aufweist, die jeweils an den Enden des Elementes (11) befestigt und mit zusammenwirkenden Schraubengewinden (15a, 16a) versehen sind, die die relative Bewegung der Bauteile und das Verdrehen des Elementes von Hand gestatten, aber eine derartige Bewegung unter dem Drehmoment verhindern, das durch das Element dadurch ausgeübt wird, um das vorher erwähnte Verriegelungsmittel (15a, 16a) bereitzustellen.

12. Vorrichtung nach einem der vorhergehenden Ansprüche, die mit einem Mittel versehen ist, das funktionsfähig ist, um Daten betreffs einer Bohrung und/oder betreffs des Betriebes der Vorrichtung zu erhalten, während sie sich durch die Bohrung bewegt.

13. Vorrichtung nach einem der vorhergehenden Ansprüche, die mit Flügeln versehen ist, die ausgebildet sind, um bei Benutzung die Vorrichtung durch eine Bohrung anzutreiben, und um die Vorrichtung um ihre Achse unter der Schwungkraft des Fluids zu drehen, das durch die Bohrung fließt.

## Revendications

1. Appareil permettant de nettoyer la surface d'un puits de forage, qui comprend un ou plusieurs élément(s) de nettoyage (12 ; 27) destiné(s) à venir en contact avec la surface d'un puits de forage lorsque l'appareil se déplace le long du puits de forage, et un moyen servant à déplacer l'élément ou les éléments de nettoyage vers l'intérieur et vers l'extérieur afin de pourvoir aux besoins de puits de forage de différentes tailles ; dans lequel ledit moyen comprend un élément allongé (11) pouvant être déformé de manière résiliente, de forme hélicoïdale ou essentiellement hélicoïdale, qui est agencé pour effectuer ledit déplacement vers l'intérieur et vers l'extérieur desdits éléments de nettoyage par torsion, extension ou contraction d'au moins une partie de l'élément avec pour résultat une augmentation ou une diminution du diamètre de la au moins une partie de l'élément et un support (10) s'étendant à travers l'élément, les extrémités dudit élément étant raccordées audit support, le support comprenant un moyen (13, 14, 15, 16) permettant un déplacement tournant relatif dans des directions opposées autour de l'axe de l'élément entre les extrémités de l'élément (11) afin de modifier l'espacement axial entre les extrémités de l'élément de manière à modifier de manière sélective le diamètre de la au moins une partie de l'élément, **caractérisé en ce que** le support (10) comprend un ressort à boudin (17) qui est d'un diamètre plus petit que celui de l'élément (11) et qui est disposé de manière concentrique au sein de celui-ci, l'élément et le ressort à boudin étant en sens contraire, grâce à quoi l'élément peut se courber à mesure que l'appareil se déplace à travers un puits de forage.

2. Appareil selon la revendication 1, dans lequel l'élément (11) est constitué par un ressort à boudin.

3. Appareil selon la revendication 1 ou 2, dans lequel l'élément ou les éléments de nettoyage (12 ; 27) est ou sont monté(s) sur la périphérie de l'élément (11).

4. Appareil selon l'une quelconque des revendications précédentes, dans lequel le, ou chaque, élément de nettoyage (12 ; 27) présente la forme d'une brosse.

5. Appareil selon l'une quelconque des revendications précédentes, qui présente un élément de nettoyage continu (12 ; 27).

6. Appareil selon l'une quelconque des revendications précédentes, dans lequel l'élément ou les éléments de nettoyage (12 ; 27) s'étend ou s'étendent le long de l'élément allongé (11) en un agencement hélicoïdal ou essentiellement hélicoïdal.

7. Appareil selon l'une quelconque des revendications précédentes, dans lequel l'élément ou les éléments de nettoyage (12 ; 27) est ou sont fixé(s) à l'élément (11).

8. Appareil selon l'une quelconque des revendications 1 à 6, dans lequel l'élément ou les éléments de nettoyage (12) est ou sont monté(s) en vue d'un mouvement vers l'intérieur ou vers l'extérieur par rapport à l'élément (11) et un moyen (21, 22, 23 ; 24, 25, 26) est fourni afin de réaliser ledit mouvement.

9. Appareil selon l'une quelconque des revendications précédentes, dans lequel le moyen (13, 14, 15, 16) permettant un mouvement tournant relatif dans des directions opposées autour de l'axe de l'élément (11) entre les extrémités de l'élément est agencé pour être utilisé manuellement afin de définir un diamètre prédéterminé de la au moins une partie de l'élément (11), un moyen de verrouillage (15a, 16a) étant fourni pour verrouiller l'élément en position.

10. Appareil selon l'une quelconque des revendications 1 à 8, dans lequel le moyen (13, 14, 15, 16) permettant un mouvement tournant relatif dans des directions opposées autour de l'axe de l'élément (11) entre les extrémités de l'élément est muni d'un moyen motorisé permettant de tordre, étendre ou contracter la au moins une partie de l'élément (11).

11. Appareil selon la revendication 9, dans lequel le support (10) comprend deux composants coaxiaux (15, 16) fixés respectivement aux extrémités de l'élément (11) et munis de filetages coopérants (15a, 16a) permettant un déplacement relatif des composants et une torsion de l'élément à la main mais empêchant un tel déplacement lorsqu'un couple est exercé par l'élément, afin de fournir ledit moyen de verrouillage susmentionné (15a, 16a).

12. Appareil selon l'une quelconque des revendications précédentes, qui est muni d'un moyen pouvant servir à obtenir des données concernant un puits de forage et/ou à faire fonctionner l'appareil lorsqu'il se déplace à travers le puits de forage.

13. Appareil selon l'une quelconque des revendications précédentes, qui est muni de ailettes agencées pour, en cours d'utilisation, propulser l'appareil à travers un puits de forage et faire tourner l'appareil autour de son axe sous l'effet d'un fluide circulant à travers le puits de forage.
